# EUROPEAN PATENT APPLICATION

(11) **EP 2 735 272 A2**
(43) Date of publication of application: **28.05.2014**
(21) Application number: 13193996.9
(22) Date of filing: 22.11.2013
(51) Int. Cl.: A61B 8/08, A61B 8/00

(54) **Ultrasound system and method for providing biometric information of fetus**

(30) Priority: 23.11.2012 KR 20120133988
(71) Applicant: Samsung Medison Co., Ltd., Gangwon-do 250-870 (KR)
(72) Inventor: Yoo, Jun-sang, Gangwon-do (KR); Kim, Sung-yoon, Gangwon-do (KR)
(74) Representative: Lorenz, Markus

(57) **Abstract**

Provided are an ultrasound system and method for providing method for providing biometric information of a fetus, i.e., graphical representation of fetal biometric information. The ultrasound system includes: an ultrasound data acquisition unit that acquires ultrasound data of a living body including a fetus; a storage unit for storing reference information corresponding to each of a plurality of measurement parameters; and a processor that generates an ultrasound image by using the ultrasound data, performs biometric measurement based on the ultrasound image, produces biometric measurement information including biometric measurement parameters of the fetus and their corresponding measurement values, calculates the measurement values as a percentile rank or percent based on the reference information, and creates a graph that represents the biometric measurement information by using the percentile rank or percent.

## Description

### RELATED APPLICATIONS

This application claims the benefit of Korean Patent Application No. 10-2012-0133988, filed on November 23, 2012, in the Korean Intellectual Property Office, the disclosure of which is incorporated herein in its entirety by reference.

### BACKGROUND

### 1. Field

One or more embodiments of the present invention relate to an ultrasound system, and more particularly, to an ultrasound system and method for providing biometric information of a fetus, i.e., graphical representation of fetal biometric information.

### 2. Description of the Related Art

Due to its non-invasive and non-destructive nature, an ultrasound system has been widely used in the medical field that requires information about the inside of living bodies. The ultrasound system also plays a critical role in the medical profession since it can provide real-time, high-resolution images of tissue of a living body to a doctor without the need for a surgical procedure that directly incises the living body for observation.

The ultrasound system transmits ultrasound signals to a living body including an object (e.g., a fetus) and receives ultrasound echo signals reflected from the living body to create an ultrasound image of the living body. The ultrasound system performs fetal biometric measurements by using an ultrasound image to create biometric information. The biometric information includes measurement information such as biparietal diameter, head circumference, abdominal circumference, femur length, arm length, leg length, and nuchal translucency (NT).

According to a conventional method, fetal measurement information is expressed in a report as a percentile for a given week of pregnancy or as a position (i.e., point) on a graph corresponding to a measurement value. In this case, a user may determine the result of measurement based on various measurement values. Thus, the conventional method may require time to understand measurement results by seeing measured values, thereby causing inconvenience to the user.

### SUMMARY

One or more embodiments of the present invention include an ultrasound system and method for providing a graph representing biometric measurement information of a fetus by a percentile rank or percent, wherein the biometric measurement information is created by performing biometric measurement on an ultrasound image.

Additional aspects will be set forth in part in the description which follows and, in part, will be apparent from the description, or may be learned by practice of the presented embodiments.

According to one or more embodiments of the present invention, an ultrasound system includes: an ultrasound data acquisition unit that acquires ultrasound data of a living body including a fetus; a storage unit for storing reference information corresponding to each of a plurality of measurement parameters; and a processor that generates an ultrasound image by using the ultrasound data, performs biometric measurement based on the ultrasound image, produces biometric measurement information including biometric measurement parameters of the fetus and their corresponding measurement values, calculates the measurement values as a percentile rank or percent based on the reference information, and creates a graph that represents the biometric measurement information by using the percentile rank or percent..

According to one or more embodiments of the present invention, a method of providing biometric information includes: acquiring ultrasound data of a living body including a fetus; generating an ultrasound image by using the ultrasound data; performing biometric measurement based on the ultrasound image to produce biometric measurement information including biometric measurement parameters of the fetus and their corresponding measurement values; calculating the measurement values as a percentile rank or percent based on reference information stored in a storage unit for storing the reference information corresponding to each of a plurality of measurement parameters; and creating a graph that represents the biometric measurement information of the fetus by using the percentile rank or percent.

The ultrasound system and method for providing biometric information of a fetus according to embodiments of the present invention allows graphic representation of measurement values for a plurality of fetal measurement parameters, thereby providing convenience for a user to identify the overall status of the fetus at first sight.

The level of risk for each of the fetal measurement parameters may also be represented by colors, thereby enabling a user to quickly understand the status of the fetus.

Furthermore, percentile ranks or percents of previous measurement values for an expected week of pregnancy, which are within the margin of error, are displayed simultaneously, thereby enabling a user to conveniently identify the status of the fetus.

In addition, previous biometric measurement information may be expressed as a percentile rank or percent by using recent biometric measurement information as reference, thereby allowing visualization of a fetal growth for a plurality of parameters.

### BRIEF DESCRIPTION OF THE DRAWINGS

These and/or other aspects will become apparent and more readily appreciated from the following description of the embodiments, taken in conjunction with the accompanying drawings of which:
FIG. 1 is a block diagram showing a configuration of an ultrasound system according to an exemplary embodiment of the present invention;
FIG. 2 is a block diagram showing a configuration of an ultrasound data acquisition unit in the ultrasound system of FIG. 1;
FIG. 3 is a flowchart of a process of providing biometric information of a fetus as a graph according to an exemplary embodiment of the present invention;
FIG. 4 is an exemplary diagram of graphical representation according to an exemplary embodiment of the present invention;
FIG. 5 is an exemplary diagram illustrating information about risk levels for a percentile rank or percent according to an exemplary embodiment of the present invention;
FIGS. 6 and 7 are exemplary diagrams illustrating a normal range for a given week of pregnancy according to an exemplary embodiment of the present invention;
FIGS. 8 through 10 are exemplary diagrams of graphs that represent measurement values in other biometric measurement information as percentile ranks or percents, by using biometric measurement information for a particular week of pregnancy as reference; and
FIG. 11 illustrates an example in which a graph is displayed on an ultrasound system according to an exemplary embodiment of the present invention.

### DETAILED DESCRIPTION

Reference will now be made in detail to embodiments, examples of which are illustrated in the accompanying drawings, wherein like reference numerals refer to the like elements throughout. In this regard, the present embodiments may have different forms and should not be construed as being limited to the descriptions set forth herein. Accordingly, the embodiments are merely described below, by referring to the figures, to explain aspects of the present description. Expressions such as "at least one of," when preceding a list of elements, modify the entire list of elements and do not modify the individual elements of the list.

Hereinafter, exemplary embodiments of the present invention will be described with reference to the accompanying drawings.

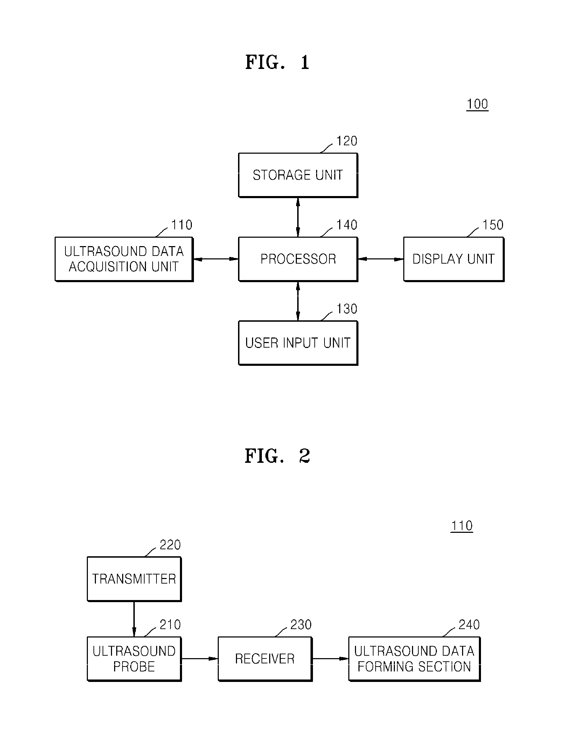

FIG. 1 is a block diagram showing a configuration of an ultrasound system 100 according to an exemplary embodiment of the present invention. Referring to FIG. 1, the ultrasound system 100 according to the present embodiment includes an ultrasound data acquisition unit 110, a storage unit 120, a user input unit 130, a processor 140, and a display unit 150.

The ultrasound data acquisition unit 110 acquires ultrasound data corresponding to an ultrasound image of a living body including an object such as a fetus. The ultrasound image includes a brightness (B) mode image, but is not limited thereto. The ultrasound data includes radio frequency (RF) data, but is not limited thereto.

FIG. 2 is a block diagram showing a configuration of the ultrasound data acquisition unit 110. Referring to FIG. 2, the ultrasound data acquisition unit 110 includes an ultrasound probe 210, a transmitter 220, a receiver 230, and an ultrasound data forming section 240.

The ultrasound probe 210 includes a plurality of transducer elements (not shown) that convert electrical signals into ultrasound signals, and vice versa. The ultrasound probe 210 is configured to transmit an ultrasound signal to a living body and receive an ultrasound echo signal reflected from the living body to generate an electrical signal (hereinafter referred to as a "reception signal"). The reception signal is an analog signal. The ultrasound probe 210 includes a convex probe, a linear probe, and a three-dimensional (3D) probe.

The transmitter 220 controls the transmission of an ultrasound signal. The transmitter 220 also produces an electrical signal (hereinafter referred to as a "transmission signal") that is used to obtain an ultrasound image in consideration of the transducer elements and a focal point. Thus, upon receipt of the transmission signal from the transmitter 220, the ultrasound probe 210 converts the transmission signal into an ultrasound signal, transmits the ultrasound signal to a living body, and creates a reception signal based on an ultrasound echo signal reflected from the living body.

The receiver 230 performs analog-to-digital conversion on the reception signal provided by the ultrasound probe 210 to produce a digital signal. The receiver 230 also performs reception beamforming on the digital signal in consideration of the transducer elements and a focal point to create a focused reception signal. Since the reception beamforming may be performed by using various known methods, a detailed description thereof is omitted here.

The ultrasound data forming section 240 creates ultrasound data corresponding to an ultrasound image by using the focused reception signal provided by the receiver 230. The ultrasound data forming section 240 may also perform various signal processings, such as gain control, needed to form ultrasound data, on the focused reception signal.

Although the ultrasound data acquisition unit 110 has been described to transmit an ultrasound signal to a living body and receives an ultrasound echo signal reflected from the living body to acquire ultrasound data corresponding to an ultrasound image, the ultrasound data acquisition unit 110 may acquire ultrasound data from an external or internal device (not shown) connected to the ultrasound system 100 in a wired or wireless manner.

Referring back to FIG. 1, the storage unit 120 stores ultrasound data acquired by the ultrasound data acquisition unit 110 as well as reference information corresponding to a plurality of measurement parameters. In the present embodiment, the measurement parameters are used for measuring an object (i.e., a fetus) and includes biparietal diameter (BPD), head circumference (HC), femur length (FL), abdominal circumference (AC), expected fetal weight (EFW), Crown-Rump Length (CRL), a heart rate, and a ratio between two of the measurement parameters. The measurement parameters may also include measurement parameters of a fetal brain, such as OccipitoFrontal Diameter (OFD), Anterior Cerebral Ventricule Diameter (Va), Posterior Cerebral Ventricle Diameter (Vp), Transverse Cerebellar Diameter (TCD), and Cisterna Magna (CM). The measurement parameters may further include cardiac biometric measurement parameters such as Cardiac Circumference, Thoracic Circumference, Left Ventricular Diameter, Right Ventricular Diameter, Aortic Valve Annulus Diameter, and Pulmonary Valve Annulus Diameter. However, the measurement parameters are not limited thereto. The reference information includes reference values needed for converting measurement values for measurement parameters to percentile ranks or percents. Since various known types of information such as a reference table may be used as the reference information, a detailed description thereof is omitted here.

For one example, the storage unit 120 may store a mapping table for providing reference information corresponding to each of the plurality of measurement parameters. Alternatively, the storage unit 120 may store a mapping table for providing a plurality of pieces of reference information corresponding to each of the measurement parameters. For another example, the storage unit 120 may store a mapping table for providing reference information corresponding to each of the measurement parameters for each week of pregnancy. Alternatively, the storage unit 120 may store a mapping table for providing a plurality of pieces of reference information corresponding to each of the measurement parameters.

The storage unit 120 also stores normal range information including a normal range of a percentile rank or percent for each week of pregnancy.

The user input unit 130 receives user input information. In the present embodiment, the user input information includes first input information needed for selecting a measurement parameter to be represented by a graph among the plurality of measurement parameters. The user input information also includes second input information needed for selecting reference information from the selected measurement parameter. The user input information further includes third input information needed for selecting the number of weeks of pregnancy. The user input unit 130 includes a control panel, a track ball, a touch screen, a keyboard, and a mouse.

The processor 140 is connected to the ultrasound data acquisition unit 110, the storage unit 120, and the user input unit 130. The processor 140 includes a central processing unit (CPU), a microprocessor, and a graphic processing unit (GPU).

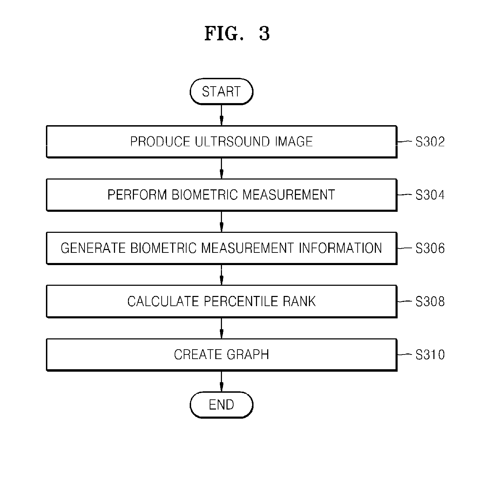

FIG. 3 is a flowchart of a process of providing biometric information of a fetus as a graph, according to an exemplary embodiment of the present invention. Referring to FIG. 3, the processor 140 produces an ultrasound image by using ultrasound data provided by the ultrasound data acquisition unit 110 (S302).

The processor 140 performs biometric measurement on the ultrasound image (S304) and creates biometric measurement information including fetal biometric measurement parameters and their corresponding measurement values (S306). Since fetal biometric parameters may be measured by using various known methods, a detailed description thereof is omitted here. The biometric measurement information may be stored in the storage unit 120.

The processor 140 calculates a measurement value in the biometric measurement information as a percentile rank or percent, based on reference information stored in the storage unit 120 (S308). In the present embodiment, the processor 140 calculates a percentile rank or percent of the measurement value contained in the biometric measurement information by using a reference value in the reference information. Since the percentile rank or percent may be calculated by using various known methods, a detailed description thereof is omitted here.

The processor 140 may selectively inquire the storage unit 120, select a measurement parameter corresponding to first input information provided by the user input unit 130, and extract reference information about the selected measurement parameter from the storage unit 120. That is, the processor 140 may select a measurement parameter corresponding to the first input information from the biometric measurement information and extract a measurement value corresponding to the selected measurement parameter from biometric measurement information. The processor 140 may calculate a percentile rank or percent of the measurement value by using the extracted reference information.

The processor 140 may selectively inquire the storage unit 120 and extract reference information that satisfy first input information and second input information provided by the user input unit 130 from the storage unit 120. The processor 140 may then extract a measurement value that satisfies the first input information and the second input information from the biometric measurement information. The processor 140 may calculate a percentile rank or percent of the measurement value by using the extracted reference information.

The processor 140 may selectively inquire the storage unit 120, select the number of weeks of pregnancy corresponding to the third input information provided by the user input unit 130, and extract a measurement parameter and reference information corresponding to the selected number of weeks of pregnancy from the storage unit 120. The processor 140 may then calculate a percentile rank or percent of the measurement value by using the extracted measurement parameter and reference information.

The processor 140 may selectively inquire the storage unit 120 and extract reference information that satisfy third input information and first input information provided by the user input unit 130 from the storage unit 120. The processor 140 may then select a measurement parameter corresponding to the first input information from the biometric measurement information and extract a measurement value corresponding to the selected measurement parameter therefrom. The processor 140 may calculate a percentile rank or percent of the measurement value by using the extracted reference information.

The processor 140 may selectively inquire the storage unit 120 and extract from the storage unit 120 reference information that satisfy third input information, first input information, and second input information provided by the user input unit 130. The processor 140 may then extract a measurement value that satisfies the first input information and the second input information from the biometric measurement information. The processor 140 may calculate a percentile rank or percent of the measurement value by using the extracted reference information.

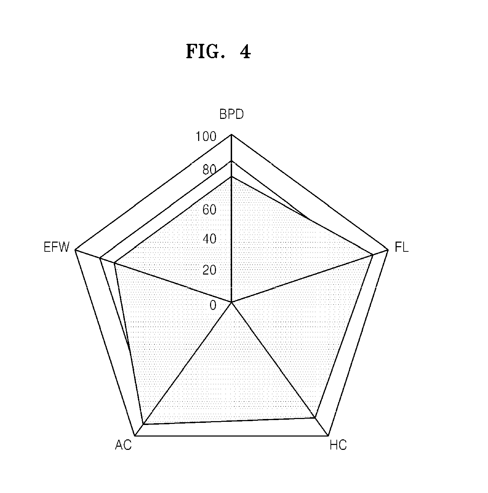

The processor 140 creates a graph that represents the biometric measurement information of a fetus by using the calculated percentile rank or percent (S310). For example, as shown in FIG. 4, the processor 140 may create a graph representing biometric measurement information of a fetus by using a percentile rank or percent for each of the measurement parameters (BPD, EFW, AC, HC, and FL).

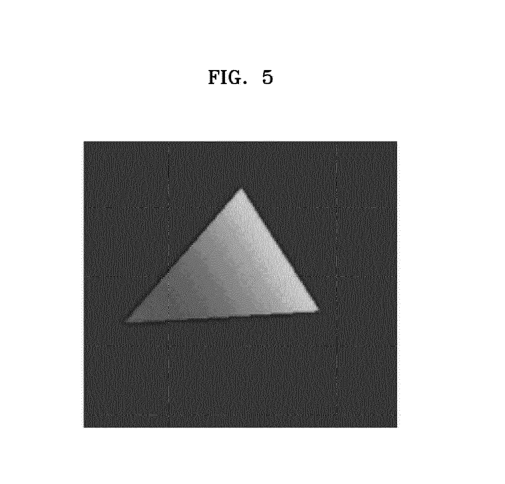

As shown in FIG. 5, the processor 140 may selectively create risk level information that represents a risk level for a percentile rank or percent by colors.

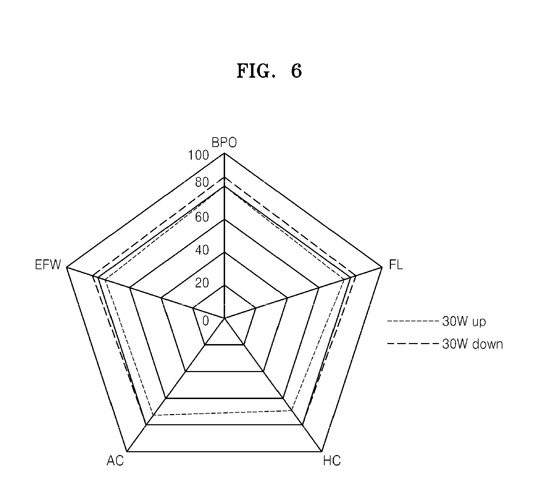

In addition, as shown in FIG. 6, the processor 140 may selectively create first normal range information that represents a normal range for a given week of pregnancy by a line based on normal range information stored in the storage unit 120. For example, for a 30 week old fetus, a normal range may be an interval between dashed lines indicating 30W up and 30W down.

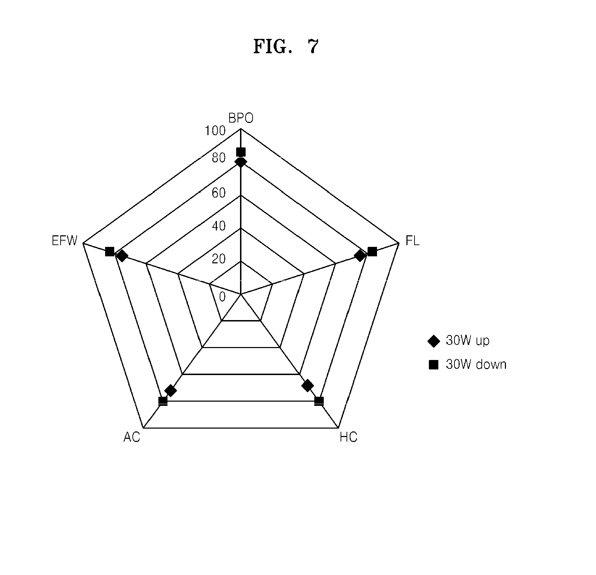

Furthermore, as shown in FIG. 7, the processor 140 may selectively create second normal range information that represents a normal range for a given week of pregnancy by dots based on normal range information stored in the storage unit 120. For example, for a 30 week old fetus, a normal range may be an interval between dots indicating 30W up and 30W down.

The processor 140 may selectively create a graph that represents a measurement value in other biometric measurement information as a percentile rank or percent by using biometric measurement information for a particular week of pregnancy as reference.

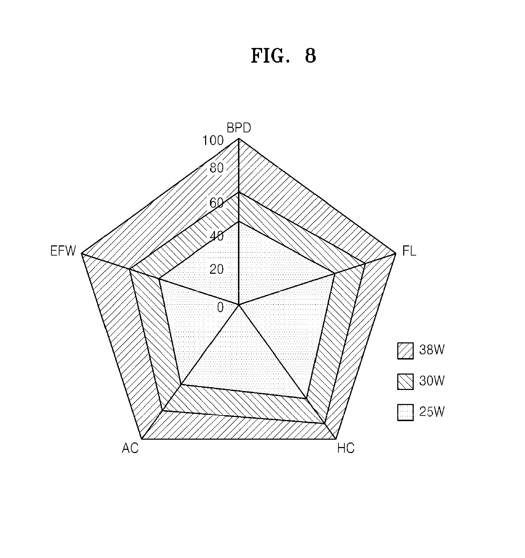

For one example, as shown in FIG. 8, the processor 140 may set the last biometric measurement information 38W as reference information and create a graph that represents measurement values in pieces of biometric measurement information 30W and 25W for previous weeks of pregnancy as percentile ranks or percents simultaneously with those of the last biometric measurement information 38W.

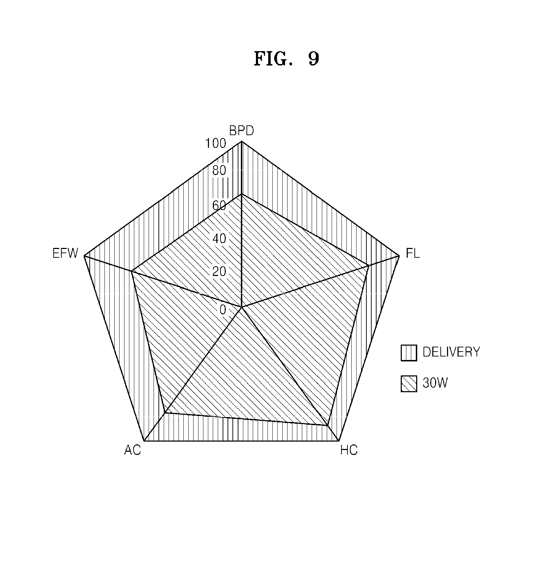

For another example, as shown in FIG. 9, the processor 140 may set biometric measurement information obtained immediately prior to delivery as reference information and create a graph that represents a measurement value in biometric measurement information 30W as a percentile rank or percent by using the reference information.

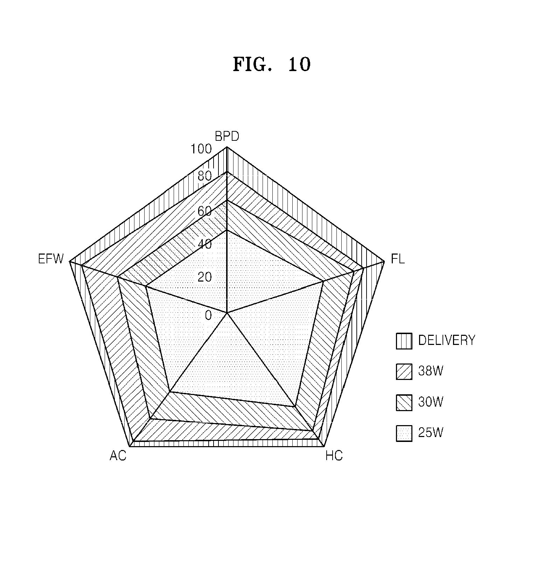

Alternatively, as shown in FIG. 10, the processor 140 may set biometric measurement information obtained immediately prior to delivery as reference information and create a graph that represents measurement values in pieces of previous biometric measurement information 38W, 30W, and 25W as percentile ranks or percents by using the reference information. In this case, the biometric measurement information obtained immediately prior to the delivery may include expected measurement values.

While the processor 140 creates a radial graph by biometric measurement information, the present invention is not limited thereto. For example, the processor 140 may create a longitudinal or transverse bar graph, a graph of broken line, a pie chart, an area chart, a box chart, or a surface chart.

Referring back to FIG. 1, the display unit 150 displays the ultrasound image as well as the graph generated by the processor 140.

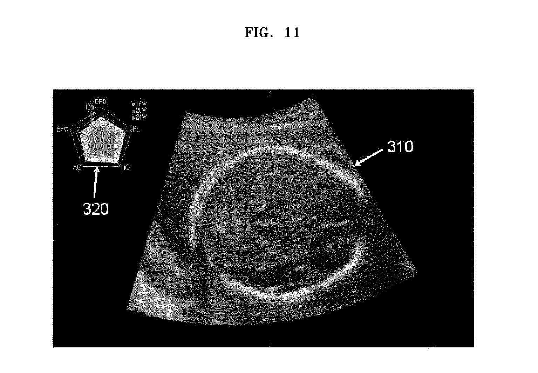

FIG. 11 illustrates an example in which a graph 320 is displayed on an ultrasound system 100 according to an exemplary embodiment of the present invention. Referring to FIG. 11, the ultrasound system 100 is configured to display an ultrasound image 310 of a cross-section of a fetal brain on a display unit 150 and perform a biometric measurement on a fetus based on the displayed ultrasound image 310. As illustrated in FIG. 11, the ultrasound system 100 may provide biometric measurement information of the fetus as the graph 320, and biometric measurement parameters may include BPD, FL, HC, AC, and EFW.

While one or more embodiments of the present invention have been described with reference to the figures, it will be understood by those of ordinary skill in the art that various changes in form and details may be made therein without departing from the spirit and scope of the present invention as defined by the following claims.

## Claims

1. An ultrasound system comprising
an ultrasound data acquisition unit that acquires ultrasound data of a living body including a fetus;
a storage unit for storing reference information corresponding to each of a plurality of measurement parameters; and
a processor that generates an ultrasound image by using the ultrasound data, performs biometric measurement based on the ultrasound image, produces biometric measurement information including biometric measurement parameters of the fetus and their corresponding measurement values, calculates the measurement values as a percentile rank or percent based on the reference information, and creates a graph that represents the biometric measurement information by using the percentile rank or percent.

2. The ultrasound system of claim 1, further comprising a user input unit for receiving at least one of first input information needed for selecting at least one of the plurality of measurement parameters and second input information needed for selecting reference information about the at least one measurement parameter selected in response to the first input information,
wherein the processor creates a graph that represents measurement information corresponding to the at least one measurement parameter.

3. The ultrasound system of claim 2, wherein the processor inquires the storage unit to select a measurement parameter corresponding to the first input information, extracts reference information corresponding to the selected measurement parameter from the storage unit, extracts a measurement value corresponding to the selected measurement parameter from the biometric measurement information, and calculates the percentile rank or percent of the measurement value by using the extracted reference information.

4. The ultrasound system of claim 2, wherein the processor inquires the storage unit to extract reference information corresponding to the second input information from the storage unit, extracts a measurement value corresponding to the selected measurement parameter from the biometric measurement information, and calculates the percentile rank or percent of the measurement value by using the extracted reference information.

5. The ultrasound system of claim 1, wherein the storage unit further stores reference information corresponding to each of the plurality of measurement parameters of the fetus for each week of pregnancy.

6. The ultrasound system of claim 5, further comprising a user input unit for receiving at least one of first input information needed for selecting at least one of the plurality of measurement parameters and third input information needed for selecting the number of weeks of pregnancy for the selected measurement parameter.

7. The ultrasound system of claim 6, wherein the processor inquires the storage unit to extract reference information corresponding to the third input information, extracts a measurement value corresponding to the selected measurement parameter from the biometric measurement information, and calculates the percentile rank or percent of the measurement value by using the extracted reference information.

8. The ultrasound system of claim 1, wherein the storage unit further stores normal range information including a normal range of the percentile rank or percent for each week of pregnancy.

9. A method of providing biometric information, the method comprising:
acquiring ultrasound data of a living body including a fetus;
generating an ultrasound image by using the ultrasound data,
performing biometric measurement based on the ultrasound image to produce biometric measurement information including biometric measurement parameters of the fetus and their corresponding measurement values;
calculating the measurement values as a percentile rank or percent based on reference information stored in a storage unit for storing the reference information corresponding to each a plurality of measurement parameters; and
creating a graph that represents the biometric measurement information of the fetus by using the percentile rank or percent.

10. The method of claim 9, prior to the calculating of the measurement values as a percentile rank or percent, further comprising receiving at least one of first input information needed for selecting at least one of the plurality of measurement parameters and second input information needed for selecting reference information about the at least one measurement parameter selected in response to the first input information,
wherein in the creating of the graph, the graph is created so as to represent measurement information corresponding to the at least one measurement parameter

11. The method of claim 10, wherein the calculating of the measurement values as a percentile rank or percent comprises:
inquiring the storage unit to select a measurement parameter corresponding to the first input information;
extracting reference information corresponding to the selected measurement parameter from the storage unit;
extracting a measurement value corresponding to the selected measurement parameter from the biometric measurement information; and
calculating the percentile rank or percent of the measurement value by using the extracted reference information.

12. The method of claim 10, wherein the calculating of the measurement values as a percentile rank or percent comprises:
inquiring the storage unit to extract reference information corresponding to the second input information from the storage unit;
extracting a measurement value corresponding to the selected measurement parameter from the biometric measurement information; and
calculating the percentile rank or percent of the measurement value by using the extracted reference information.

13. The method of claim 9, wherein the storage unit further stores reference information corresponding to each of the plurality of measurement parameters of the fetus for each week of pregnancy.

14. The method of claim 13, prior to the calculating of the measurement values as a percentile rank or percent, further comprising receiving at least one of first input information needed for selecting at least one of the plurality of measurement parameters and third input information needed for selecting the number of weeks of pregnancy for the selected measurement parameter.

15. The method of claim 14, wherein the calculating of the measurement value as a percentile rank or percent comprises:
Inquiring the storage unit to extract reference information corresponding to the third input information;
extracting a measurement value corresponding to the selected measurement parameter from the biometric measurement information; and
calculating the percentile rank or percent of the measurement value by using the extracted reference information.
